# EUROPÄISCHE PATENTSCHRIFT

(11) **EP 1 690 087 B1**
(45) Veröffentlichungstag und Bekanntmachung des Hinweises auf die Patenterteilung: **09.05.2018**
(21) Anmeldenummer: 04803503.4
(22) Anmeldetag: 03.12.2004
(51) Int. Cl.: G01N 33/487, B01L 3/00

(54) **BESCHICHTETE TESTELEMENTE**
COATED TEST ELEMENTS
ELEMENTS DE TEST REVETUS

(30) Priorität: 04.12.2003 DE 10356752
(43) Veröffentlichungstag der Anmeldung: 16.08.2006
(73) Patentinhaber: Roche Diabetes Care GmbH, 68305 Mannheim (DE); F. Hoffmann-La Roche AG, 4070 Basel (CH)
(72) Erfinder: PACHL, Rudolf, 67158 Ellerstadt (DE); HOFERER, Jürgen, 76133 Karlsruhe (DE); BABIC, Branislav, 68167 Mannheim (DE); FRANK, Martin, 67246 Dirmstein (DE); SCHMID, Wilfried, 68165 Mannheim (DE)
(74) Vertreter: Weickmann & Weickmann PartmbB
(86) Internationale Anmeldenummer: PCT/EP2004/013782
(87) Internationale Veröffentlichungsnummer: WO 2005/054845

(56) Entgegenhaltungen:
- EP-A- 0 010 456
- EP-A- 0 502 691
- EP-A1- 1 362 551
- WO-A-02/49763
- WO-A-97/46887
- DE-A1- 19 942 928
- DE-A1- 19 944 256
- US-B1- 6 284 550

## Beschreibung

Die Erfindung betrifft beschichtete Testelemente, insbesondere Testelemente mit einem Kapillarspalt, die zumindest in dem den Kapillarspalt umgebenden Bereich eine hydrophobe strukturierte Beschichtung aufweisen.

Testelemente für diagnostische Vorrichtungen, z.B. Blutzuckermesssysteme, werden vielfach von den Patienten selbst angewendet. Die Anwendung umfasst im Allgemeinen das Aufbringen einer Probe von Körperflüssigkeit, z.B. Blut, auf das Testelement, das Einführen des Testelements in eine Messvorrichtung und die Bestimmung eines Analyten aus der Körperflüssigkeit in der Messvorrichtung. Dabei war bisher der Verbleib von überschüssigem Probenmaterial, z.B. Blut, alleine dem Anwender überlassen. Bei Entsorgung des Testelements konnte dieses Probenmaterial je nach Entsorgungsort eine ernste Verschmutzungsgefahr und ein Hygieneproblem darstellen. Besonders relevant war dieses Problem bei Teststreifen mit Kapillarspalt, bei denen es oftmals zum Anhaften von Blut an der Außenseite der Kapillare kam.

Der aktuelle Trend bei Blutzuckermesssystemen läuft auf eine zunehmende Integration der Handhabungsschritte und Systemeinzelteile hinaus. Dabei werden die Testelemente oftmals in bzw. durch die Messvorrichtung gezogen. Auf dem Weg des Testelements durch die Vorrichtung stellt jedes außen am Testelement anhaftende Probenmaterial ein Verschmutzungsrisiko des Messgeräts dar. Insbesondere besteht dieses Verschmutzungsrisiko des Messgeräts bei der Remagazinierung der Teststreifen. Hierbei werden Teststreifen, nachdem eine Probenaufgabe an einem Teststreifen erfolgt ist, innerhalb des Messgerätes transportiert. Ein Verschmutzungsrisiko besteht folglich zum einen während des Testelementtransports, nachdem eine Probe aufgegeben wurde, und zum anderen durch die Lagerung bereits verwendeter Testelemente innerhalb des Magazins. Hierbei besteht das Risiko, dass getrocknetes Blut vom Testelement abfällt, sodass eine Verschmutzung des Magazingehäuses bzw. eine Kontamination noch zur Verwendung vorgesehener Testelemente bedingt wird.

Ein weiteres Problem ist, dass an der Außenseite von Testelementen angetrocknetes Probenmaterial abkrümeln und Geräteteile, Optik und die Umwelt kontaminieren kann.

WO 97/46887 beschreibt eine Remagazinierung von Küvetten bzw. Testelementen. Überflüssige Probenreste, z.B. Blutreste, werden dabei durch ein vorstehendes Ende des Testelements aufgefangen, sodass Kontaminationen des Messgerätes bei der Remagazinierung vermieden werden können. Diese Maßnahme ist jedoch konstruktiv aufwändig und führt nicht zu einer zuverlässigen Vermeidung von Verschmutzungen bei der Remagazinierung. Die WO 99/29429 beschreibt ein analytischesTestelement zur Bestimmung eines Analyten in einer Flüssigkeit, umfassend einen inerten Träger, eine Aufgabezone für Probenmaterial, eine Nachweiszone zur Bestimmung des Analyten, und einen Kanal oder Spalt zum Transport von Flüssigkeit von der Aufgabezone zur Nachweiszone, wobei es zumindest in einem Bereich um die Aufgabezone eine hydrophobe strukturierte Oberfläche aufweist.

Es besteht deshalb ein Bedarf nach Testelementen und Testverfahren, bei denen eine Verschmutzung durch Probenmaterial, insbesondere bei der Remagazinierung, möglichst weitgehend vermieden werden kann. Versuche zur hydrophoben Beschichtung von Testelementen mit Tensiden und Wachsen, mit Teflonfolien und Spray sowie mit Silikon-haltigen Hydrophobisierungsmitteln erwiesen sich jedoch weitgehend als erfolglos.

Bei Beschichtung von Testelementen mit hydrophoben strukturierten Oberflächen, welche auch unter dem Namen Lotus-Oberflächen bekannt sind, wurde überraschenderweise gefunden, dass eine Verschmutzung mit Probenmaterial, insbesondere mit Blut, weitestgehend ausgeschlossen werden kann. Insbesondere bei Testelementen, die eine Kanalöffnung bzw. einen Spalt zur Aufnahme und zum Transport von Probenmaterial aufweisen, konnte gezeigt werden, dass durch Lotus-Oberflächen eine ausschließliche Benetzung der Kanalöffnung bzw. des Spaltes erzielt wird

Darüber hinaus hat die Beschichtung mit Lotus-Oberflächen den Vorteil, dass eine genaue Dosierung und somit eine Reduktion des Probenvolumens möglich ist, wie es im Folgenden ausführlich dargelegt wird.

Insbesondere eignet sich die Lotus-Oberflächen-Beschichtung für Testelemente mit hydrophiler Kapillarstruktur, da bei derartigen Testelementen mit einer Verschmutzung des Randbereiches der Testelemente und somit des Messgerätes gerechnet werden muss. Bei derartigen Systemen ragen die Testelemente während der Blutaufgabe zunächst aus dem Gerät heraus. Das Blut wird von dem Benutzer auf dem aus dem Gerät hervorstehenden Ende des Testelements, an dem die Öffnung der Kapillare angeordnet ist, aufgetragen. Bei Beschichtung des Testelements mit einer Lotus-Oberfläche, zumindest im Bereich der Öffnung, wird überschüssiges Blut entweder in die Kapillare eingesaugt oder tropft am Testelementrand ab, sodass ausschließlich der Kapillarspalt des Testelements benetzt wird, wobei an dem den Kapillarspalt umgebenden Bereich des Testelements kein Blut anhaftet.

Ein Gegenstand der Erfindung ist somit ein analytisches Testelement zur Bestimmung eines Analyten in einer Flüssigkeit entsprechend Anspruch 1. Bei hydrophob strukturierten Oberflächen bzw. Lotus-Effekt-Oberflächen handelt es sich um selbstreinigende Oberflächen, die Erhebungen aufweisen, wobei der mittlere Abstand zwischen Erhebungen vorzugsweise im Bereich von 50 nm bis 200 µm, besonders bevorzugt im Bereich von 50 nm bis 10 µm und die mittlere Höhe der Erhebungen vorzugsweise im Bereich von 50 nm bis 100 µm, besonders bevorzugt im Bereich von 50 nm bis 10 um, liegt. Weiterhin zeichnen sich Lotus-Effekt-Oberflächen vorzugsweise durch eine Oberflächenenergie von weniger als vorzugsweise 20 mN/m sowie erfindungsgemäß durch einen Kontaktwinkel von ≥ 120° und bis zu 160° gegenüber wässrigen Systemen auf. Zumindest die Erhebungen bestehen aus hydrophoben Materialen, beispielsweise Nanopartikeln mit hydrophoben Eigenschaften. Bevorzugte Beispiele für Oberflächen mit Lotus-Effekt sind in EP-B-0 772 514, EP-B-0 933 388, EP-A-1 018 531, EP-A-1 040 874, EP-B-1 171 529, EP-A-1 249 280 und EP-A-1 249 467 beschrieben. Strukturierte hydrophobe Oberflächen mit Lotus-Effekt können, wie in den oben genannten Dokumenten beschrieben, durch viele verschiedene Methoden erzeugt werden, z.B. durch Beschichten, Tränken, Sprühen, Koextrudieren oder durch Spritzguss. Bevorzugt ist das Aufsprühen einer Suspension von hydrophoben Nanopartikein.

Besonders bevorzugt ist ein Verfahren, welches eine Fixierung der hydrophoben strukturierten Beschichtung auf der Oberfläche des Testelements beinhaltet. Dabei wird vorzugsweise zunächst eine härtbare Substanz auf die zu beschichtenden Bereiche des Testelements aufgebracht, anschließend werden hydrophobe Partikel, mit günstigerweise zerklüfteten Strukturen, auf die zu beschichtenden Bereiche aufgebracht und danach werden die Partikel durch Härten fixiert, wie in EP-A-1 249 280 beschrieben ist. Als härtbare Substanzen sind z.B. Lacke geeignet, die einfach oder/und mehrfach ungesättigte Acrylate oder/und Methacrylate oder/und Polyurethane oder/und Silikonacrylate oder/und Urethanacrylate aufweisen. Die Lacke weisen vorzugsweise hydrophobe Eigenschaften auf. Die Partikel können selbst hydrophob, z.B. pulverförmige Polymere, insbesondere halogenierte Kohlenwasserstoffe wie etwa Polytetrafluorethylen, oder hydrophobierte Partikel, z.B. hydrophobe Aerosile, sein. Gegebenenfalls kann die Hydrophobisierung der Partikel auch nach dem Fixieren auf dem Träger erfolgen. Das Fixieren der Partikel erfolgt durch Härten, beispielsweise durch thermische oder chemische Energie oder/und durch Lichtenergie. Eine durch ein solches mehrstufiges Verfahren aufgebrachte hydrophobe Schicht ist besonders stabil gegen Abrieb und mechanische Belastungen.

Durch Bereitstellung eines Testelements, das eine Lotus-Effekt-Oberfläche in einem Bereich um die Aufgabezone und insbesondere um eine Kanalöffnung bzw. einen Spalt im Bereich der Aufgabezone aufweist, wird eine erleichterte Dosierung der Probenflüssigkeit ermöglicht, da die Probenflüssigkeit automatisch zur Kanalöffnung hingeleitet und ein Anhaften an die Kanalöffnung umgebenden Bereiche des Testelements verhindert wird. Dies ist insbesondere für Diabetespatienten, bei denen es sich oft um ältere oder sehbehinderte Personen handelt, von besonderem Interesse.

Aufgrund der exakten Dosierbarkeit durch die hydrophobe strukturierte Oberfläche ist es weiterhin möglich, das Probenvolumen zu reduzieren, sodass eine schmerzarme Blutentnahme von nur noch sehr geringen Blutmengen ermöglicht wird. Darüber hinaus zeigt sich, dass insbesondere bei Remagazinierung von Testelementen eine hydrophobe Beschichtung der Testelemente vorteilhaft ist. Bei einer derartigen Remagazinierung wird das Testelement, das zunächst aus einem gegebenenfalls in die Messvorrichtung integrierten Magazin zur Probenaufgabe, z.B. zur Aufgabe von Blut, hinausgeschoben wird, anschließend wieder in dieses zurückgezogen. Bei erfindungsgemäßer Beschichtung des Testelements zeigt sich, dass eine Verschmutzung der Außenseite nicht zu befürchten ist.

Das erfindungsgemäß beschichtete analytische Testelement besitzt eine Kanalöffnung bzw. einen Spalt im Bereich der Aufgabezone, wobei die Oberfläche des Testelements zumindest um die Kanalöffnung herum eine hydrophobe Strukturierung aufweist. Gegebenenfalls kann die Oberfläche des Testelements, zumindest die Teile, die vom Träger sowie von eventuell vorhandenen Abdeckungen oder Zwischenschichten gebildet werden, vollständig mit einer hydrophob strukturierten Beschichtung belegt sein. Der Kanal ist vorzugsweise ein Kapillarkanal bzw. Kapillarspalt, d.h. ein zum kapillaren Flüssigkeitstransport zur Nachweiszone des Testelements befähigter Kanal bzw. Spalt, der neben einer Öffnung in der Aufgabezone an seinem anderen Ende gegebenenfalls eine Entlüftungsöffnung besitzen kann. Der Kanal weist in seinem Inneren vorzugsweise zumindest teilweise eine hydrophile oder hydrophil beschichtete Oberfläche auf, z.B. eine metallische oder oxidische Oberfläche.

Der Kanal- oder Spaltquerschnitt kann grundsätzlich beliebig sein. Vorzugsweise hat der Kanal bzw. Spalt einen im Wesentlichen rechteckigen Querschnitt, dessen Dimensionen durch die physikalischen Grenzen der Kapillaraktivität vorgegeben sind. Die Höhe des Kanals bzw. Spalts liegt beispielsweise für wässrige Probenflüssigkeiten in der Größenordnung von 10 bis 500 µm, bevorzugt zwischen 20 und 300 µm. Je nach gewünschtem Kanal- bzw. Spaltvolumen kann die Breite dann mehrere mm, bevorzugt 1 bis 10 mm, besonders bevorzugt 1 bis 3 mm, und die Länge bis zu einigen cm, bevorzugt 0.5 bis 5 cm, ganz bevorzugt 1 bis 3 cm, betragen.

Vorzugsweise weist die Kante des Testelements, die die Probenaufgabezone bildet, eine Aussparung im Bereich der den Kanal bzw. Spalt bildenden Fläche auf, um den Eintritt der Probenflüssigkeit in den Kanal zu erleichtern. Die Dimension der Aussparung, z.B. deren Breite, wird vorzugsweise so gewählt, dass der Durchmesser des Tropfens der Probenflüssigkeit, der auf das Testelement aufgebracht wird, geringfügig größer ist als die gewählte Dimension der Aussparung. So hat sich für ein Tropfenvolumen von 3 µl eine Breite der Aussparung von etwa 1 mm als geeignet erwiesen. Die durch die Aussparung freiliegende Fläche weist - ebenso wie der Kanal oder Spalt selbst - vorzugsweise eine hydrophile oder hydrophil beschichtete Oberfläche auf.

Desweiteren enthält das Testelement einen Teil oder alle der zur Bestimmung des Analyten notwendigen Reagenzien und gegebenenfalls Hilfsstoffe. Diese Reagenzien umfassen beispielsweise Enzyme, Enzymsubstrate, Indikatoren, Puffersalze, inerte Füllstoffe und dergleichen. Die Reagenzien sind vorzugsweise im Bereich der Nachweiszone enthalten. Die Nachweiszone kann aus einem oder mehreren Bereichen aufgebaut sein und enthält üblicherweise saugfähige Materialien, die mit den Reagenzien imprägniert sind. Beispiele für saugfähige Materialien sind Vliese, Gewebe, Gewirke oder poröse Kunststoffmaterialien, die in Form von beispielsweise Schichten vorliegen können. Bevorzugte Materialien sind Papiere oder poröse Kunststoffmaterialien wie Membranen. Besonders bevorzugt enthält die Nachweiszone offene Filme, wie sie z.B. in EP-B-0 016 387 beschrieben sind. Die Filme können aus einer oder mehreren Schichten bestehen und auf einem Träger des Testelements aufgebracht sein.

Die Nachweiszone kann außerdem über Bestandteile verfügen, die einen Ausschluss störender Probenanteile von der Nachweisreaktion erlauben und somit als Filter, beispielsweise für partikuläre Probenbestandteile wie Blutzellen, wirken. Geeignete Beispiele hierfür sind semipermeable Membranen oder Glasfaservliese, wie sie aus EP-B-0 045 476 bekannt sind.

Die Bestimmung des Analyten in der Nachweiszone kann durch optische Methoden, z.B. durch visuelle oder photometrische Bestimmung, durch elektrochemische Methoden oder andere geeignete Nachweismethoden erfolgen.

Das Testelement kann weiterhin Abdeckungen oder/und Zwischenschichten enthalten, die zusammen mit dem Träger und gegebenenfalls der Nachweiszone die Begrenzung des Probentransportkanals bzw. -spalts bilden. Die Abdeckungen und Zwischenschichten können in ihren Eigenschaften, wie z.B. Material und Beschichtung, gleich oder ähnlich dem inerten Träger sein. Besonders bevorzugt ist dabei auf der dem Kanal bzw.

Spalt hinzugewandten Seite einer Abdeckung eine flexible inerte Folie angebracht, die über die gesamte Länge der Abdeckung reicht, den Kanal bzw. Spalt auf der gesamten Breite bedeckt und die, zumindest teilweise zwischen den sich gegenüber liegenden Flächen der Abeckung und des Nachweiselements, eingeschlossen ist, sodass der kapillare Flüssigkeitstransport an der Berührungsstelle von Nachweiszone und Abdeckung nicht abreißt.

Besonders bevorzugt sind analytische Testelemente mit einem Kapillarkanal, wie sie etwa in WO 99/29429 beschrieben sind. Diese Testelemente zeichnen sich dadurch aus, dass ein zum kapillaren Flüssigkeitstransport befähigter Kanal bzw. Spalt zumindest teilweise vom Träger und Nachweiszone gebildet wird und in Richtung des kapillaren Transports von der Probenaufgabezone zumindest bis zu der einer Entlüfungsöffnung im Testelement nächstgelegenen Kante der Nachweiszone reicht und dass sich eine Aussparung in einer dem zum kapillaren Flüssigkeitstransport befähigten Kanal oder Spalt bildende Fläche, an der sich die Probenaufgabeöffnung bildenden Kante des Testelements befindet, sodass die die Probenaufgabeöffnung bildende Kante des Testelements auf einer Seite zumindest teilweise unterbrochen ist und die der Aussparung gegenüber liegende Fläche freiliegt.

Das erfindungsgemäß beschichtete Testelement ist vorzugsweise für die Aufnahme innerhalb eines Magazins vorgesehen, das ein oder mehrere Testelemente enthalten kann.

Bevorzugt ist das Testelement für eine Remagazinierung vorgesehen, d.h. zur Aufnahme in einem Magazin, das gemeinsam gebrauchte und ungebrauchte Testelemente enthalten kann, wobei ein ungebrauchtes Testelement vor Gebrauch aus dem Magazin entnommen und nach Gebrauch wieder in das Magazin rückgeführt wird. Die Entnahme oder/und Rückführung kann manuell oder automatisch erfolgen.

Das einzelne Testelement kann ein Wegwerf-Testelement oder ein mehrfach benutzbares Testelement kann. Das Magazin kann innerhalb einer Messvorrichtung angeordnet sein, die beispielsweise für einen optischen oder elektrochemischen Nachweis vorgesehen ist.

Das Testelement kann zum Nachweis beliebiger Analyten in flüssigen Probenmaterialien, insbesondere Körperflüssigkeiten wie Blut, Speichel oder Urin eingesetzt werden. Besonders bevorzugt ist die Bestimmung von Glucose in Blut. Andere bevorzugte Beispiele für die Anwendung der Testelemente sind die Koagulationsmessung oder die Messung von HBA1C.

Ein weiterer Gegenstand der vorliegenden Erfindung ist eine Messvorrichtung zur Bestimmung eines Analyten in einer Flüssigkeit, die ein erfindungsgemäß beschichtetes Testelement enthält. Die Messvorrichtung kann ein oder mehrere Magazine zur Aufnahme von einem oder mehreren Testelementen integriert enthalten. Bevorzugt ist eine Messvorrichtung mit remagazinierten Testelementen, wobei gebrauchte und ungebrauchte Testelemente gemeinsam in einem Magazin vorliegen können.

Noch ein weiterer Gegenstand der vorliegenden Erfindung ist ein Verfahren zur Bestimmung eines Analyten in einer Flüssigkeit, umfassend
- Aufbringen einer Probenflüssigkeit auf ein erfindungsgemäß beschichtetes Testelement und
- qualitatives und/oder quantitatives Bestimmen des in der Probenflüssigkeit vorhandenen Analyten.

Vorzugsweise wird ein Volumen der Probenflüssigkeit, z.B. ein Volumen von 1 bis 10 µl, auf das Testelement aufgebracht. Das Aufbringen erfolgt im Bereich der Aufgabezone des Testelements, wobei die Oberfläche zumindest im Bereich um die Aufgabezone erfindungsgemäß hydrophob strukturiert beschichtet ist, um eine Verschmutzung durch überschüssiges oder/und ungenau aufgebrachtes Probenmaterial zu vermeiden.

Die Bestimmung des Analyten erfolgt vorzugsweise in einer integrierten Messvorrichtung, wobei das Testelement von einer ersten Position in der Vorrichtung, z.B. in einem ersten Magazin, zu einer zweiten Position, z.B. einer Position zur Probenaufgabe, dann zu einer dritten Position, z.B. einer Position zur Bestimmung des Analyten und dann aus der Vorrichtung hinaus oder zu einer vierten Position, z.B. im ersten oder einem anderen Magazin, transportiert wird. Die Vorrichtung kann ein oder mehrere Magazine enthalten, die jeweils zur Aufnahme von einem oder mehreren Testelementen vorgesehen sind. Besonders bevorzugt ist eine Vorrichtung mit einem Magazin, in dem gebrauchte und ungebrauchte Testelemente gemeinsam vorliegen.

Bevorzugte Ausführungsformen der vorliegenden Erfindungen werden durch die beigefügten Figuren und Beispiele erläutert:

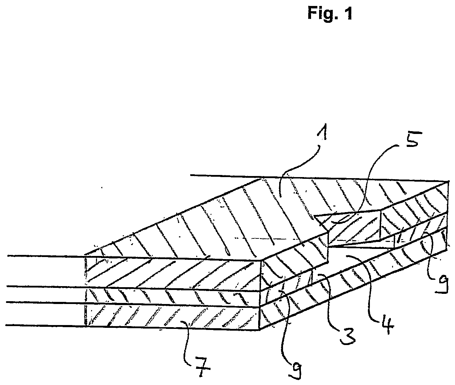

**Figur 1** zeigt eine Detailvergrößerung in perspektivischer Ansicht einer Probenaufgabezone in einer besonders bevorzugten Ausführungsform des erfindungsgemäßen Testelements. Das Testelement enthält einen Träger (1) mit einer Aussparung (5), die das Eindringen einer Probenflüssigkeit von der Probenaufgabezone (4) in einen Kapillar-aktiven Kanal (3) erleichtert, der im vorliegenden Fall vom Träger (1), einer Zwischenschicht (9) und einer Abdeckung (7) gebildet wird. Der Kanal (3) führt zur Nachweiszone des Testelements (nicht gezeigt). Die Aussparung kann neben der gezeigten Form auf jede andere, beliebige Form besitzen, die dem erfindungsgemäßen Zweck dienlich ist.

Die erfindungsgemäße Beschichtung kann auf den schraffiert gezeichneten Oberflächenbereichen des Trägers (1) der Abdeckung (7) der Zwischenschicht (9) sowie auf der nicht gezeigten Unterseite der Abdeckung (7) vorhanden sein.

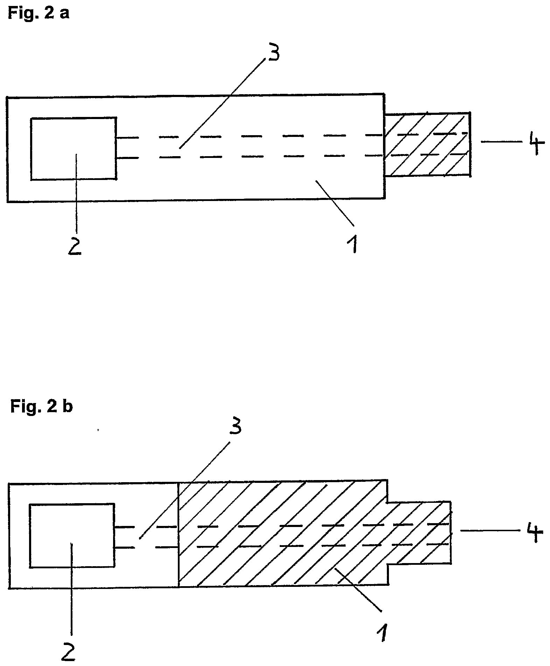

Die **Figuren 2a** **und b** zeigen beispielhaft mögliche Applikationsbereiche der erfindungsgemäßen Beschichtung auf einem Testelement. Das Testelement enthält einen Träger (1), eine Nachweiszone (2) sowie eine Probenaufgabezone (4), die über einen Kanal (3) mit der Nachweiszone (2) in Verbindung steht. Die erfindungsgemäße Beschichtung (schraffiert gezeichnet) kann -wie in Fig. 2a gezeigt - nur auf einem begrenzten Teil des Trägers im Bereich der Probenaufgabezone (4) vorliegen oder - wie in Fig. 2b gezeigt - sich über einen Großteil des Testelements erstrecken.

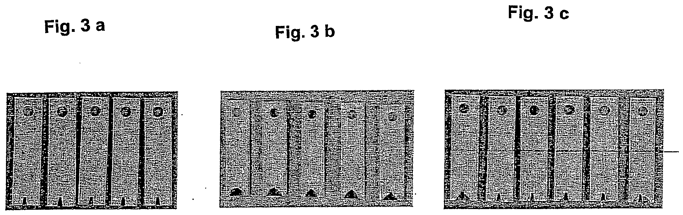

In **Figur 3** ist ein Vergleich der Blutaufnahmeeigenschaften von Testelementen mit Lotus-Effekt-Oberfläche (Fig. 3a), einer unbehandelten Oberfläche (Fig. 3b) und einer Teflon-beschichteten Oberfläche (Fig. 3c). Die Testelemente weisen eine Probenaufgabezone mit Aussparung (unten) auf, die über einen Kapillarkanal mit einer Nachweiszone in Verbindung steht.

### Beispiel

Ein gemäß WO 99/29429 hergestellte Testelement wurde auf der Außenseite mit einem Spray (Lotus-Effekt Spray, Creavis) beschichtet, durch welches die Oberfläche der Trägerfolie mit hydrophoben Nanopartikeln belegt wird, sodass eine hydrophobe Struktur mit Erhebungen entsteht. Nach Eintauchen des auf diese Weise behandelten Streifens in einen 10 µl Blutstropfen, zeigt sich kein Blut an der Außenseite des Testelements (Fig. 3a). Dagegen wurden bei einem Standard-Testelement mit gewachster Außenseite (Fig. 3b) sowie einem mit Teflonspray behandelten Testelement (Fig. 3c) starke Verschmutzungen durch Blut im Bereich der Aufgabezone gefunden.

## Patentansprüche

1. Analytisches Testelement zur Bestimmung eines Analyten in einer Flüssigkeit, umfassend
- einen inerten Träger (1),
- eine Aufgabezone für Probenmaterial (4),
- eine Nachweiszone zur Bestimmung des Analyten (2), und
- einen Kanal oder Spalt (3) zum Transport von Flüssigkeit von der Aufgabezone zur Nachweiszone,
wobei das Testelement zumindest in einem Bereich um die Aufgabezone (4) eine hydrophobe strukturierte Oberfläche aufweist, **dadurch gekennzeichnet,**
**dass** die hydrophobe strukturierte Oberfläche einen Kontaktwinkel mit wässrigen Systemen ≥ 120° aufweist.

2. Testelement nach Anspruch 1,
**dadurch gekennzeichnet,**
**dass** der Kanal oder Spalt (3) im Bereich der Aufgabezone (4) eine Öffnung aufweist und die Oberfläche des Testelements zumindest um die Öffnung herum die hydrophobe strukturierte Oberfläche aufweist.

3. Testelement nach Anspruch 1 oder 2,
**dadurch gekennzeichnet,**
**dass** der Kanal oder Spalt (3) ein Kapillarkanal oder Kapillarspalt ist.

4. Testelement nach einem der Ansprüche 1 bis 3,
**dadurch gekennzeichnet,**
**dass** der Kanal oder Spalt (3) im Inneren zumindest teilweise eine hydrophile Oberfläche aufweist.

5. Testelement nach einem der Ansprüche 1 bis 4,
**dadurch gekennzeichnet,**
**dass** der mittlere Abstand zwischen Erhebungen auf der hydrophoben strukturierten Oberfläche im Bereich von 50 nm bis 200 µm und die mittlere Höhe der Erhebungen im Bereich von 50 nm bis 100 µm liegt.

6. Testelement nach einem der Ansprüche 1 bis 5,
**dadurch gekennzeichnet,**
**dass** die hydrophobe strukturierte Oberfläche eine Oberflächenenergie von ≤ 20 mN/m aufweist.

7. Testelement nach einem der Ansprüche 1 bis 6,
**dadurch gekennzeichnet,**
**dass** die hydrophobe strukturierte Oberfläche durch Aufsprühen einer Suspension von hydrophoben Nanopartikeln erhältlich ist.

8. Testelement nach einem der Ansprüche 1 bis 7,
**dadurch gekennzeichnet,**
**dass** die hydrophobe strukturierte Oberfläche auf dem Testelement fixiert ist.

9. Testelement nach Anspruch 8,
**dadurch gekennzeichnet,**
**dass** die hydrophobe strukturierte Oberfläche durch Aufbringen einer härtbaren Substanz auf die zu beschichtenden Bereiche des Testelements, Aufbringen von hydrophoben, hydrophobisierten oder hydrophobisierbaren Partikeln auf die zu beschichtenden Bereiche und Fixieren der Partikel durch Härten erhältlich ist.

10. Testelement nach einem der Ansprüche 1 bis 9 zur Bestimmung von Glucose in Blut.

11. Verwendung des Testelements nach einem der Ansprüche 1 bis 10, zur Aufnahme innerhalb eines Magazins.

12. Verwendung des Testelements nach Anspruch 11 in einem Magazin, welches für die gemeinsame Aufnahme von gebrauchten und ungebrauchten Testelementen vorgesehen ist.

13. Verwendung eines Testelements nach Anspruch 11 oder 12 in einem Magazin, welches innerhalb einer Messvorrichtung angeordnet ist.

14. Verwendung des Testelements nach Anspruch 13, wobei die Messvorrichtung eine optische oder elektronische Messvorrichtung ist.

15. Magazin für die Aufnahme von Testelementen zur Bestimmung eines Analyten in einer Flüssigkeit, umfassend mindestens ein Testelement nach einem der Ansprüche 1 bis 10.

16. Magazin nach Anspruch 15,
**dadurch gekennzeichnet,**
**dass** es für die gemeinsame Aufnahme von gebrauchten und ungebrauchten Testelementen vorgesehen ist.

17. Messvorrichtung zur Bestimmung eines Analyten in einer Flüssigkeit,
**dadurch gekennzeichnet,**
**dass** sie mindestens ein Testelement nach einem der Ansprüche 1 bis 10 enthält.

18. Messvorrichtung nach Anspruch 17,
**dadurch gekennzeichnet,**
**dass** sie mindestens ein Magazin zur Aufnahme von einem oder mehreren der Testelemente enthält.

19. Messvorrichtung nach Anspruch 18,
**dadurch gekennzeichnet,**
**dass** das Magazin für die gemeinsame Aufnahme von gebrauchten und ungebrauchten Testelementen vorgesehen ist.

20. Verfahren zur Bestimmung eines Analyten in einer Flüssigkeit, umfassend
- Aufbringen einer Probenflüssigkeit auf ein Testelement nach einem der Ansprüche 1 bis 10, und
- qualitatives oder/und quantitatives Bestimmen des in der Probenflüssigkeit vorhandenen Analyten.

## Claims

1. Analytical test element to determine an analyte in a liquid, comprising
- an inert support (1),
- a feed-in area for sample material (4),
- an analysis area for determining the analyte (2), and
- a channel or slot (3) for transporting liquid from the feed-in area to the analysis area, in which the test element has a hydrophobically structured surface (4) at least in one area around the feed-in area,
**characterised in that**
the hydrophobically structured surface has a contact angle with aqueous systems ≥ 120°.

2. Test element in accordance with claim 1,
**characterised in that**
in the region of the feed-in area (4) the channel or slot (3) has an aperture and the surface of the test element has the hydrophobically structured surface, at least around the aperture.

3. Test element in accordance with claim 1 or 2,
**characterised in that**
the channel or slot (3) is a capillary channel or capillary slot.

4. Test element in accordance with one of claims 1 to 3,
**characterised in that**
on the interior the channel or slot (3) has at least to some extent a hydrophilic surface.

5. Test element in accordance with one of claims 1 to 4,
**characterised in that**
the average distance between elevations on the hydrophobically structured surface is in the range from 50 nm to 200 µm and the average height of the elevations in the range from 50 nm to 100 µm.

6. Test element in accordance with one of claims 1 to 5,
**characterised in that**
the hydrophobically structured surface has a surface energy of ≤ 20 mN/m.

7. Test element in accordance with one of claims 1 to 6,
**characterised in that**
the hydrophobically structured surface can be obtained by spraying a suspension of hydrophobic nano-particles.

8. Test element in accordance with one of claims 1 to 7,
**characterised in that**
the hydrophobically structured surface is fixed onto the test element.

9. Test element in accordance with claim 8,
**characterised in that**
the hydrophobically structured surface can be obtained by depositing a hardenable substance to the areas of the test element to be coated, depositing of hydrophobic, hydrophobitised or particles capable of being hydrophobitised to the areas to the coated and fixing of the particles by curing.

10. Test element in accordance with one of claims 1 to 9 for determining blood glucose.

11. Use of the test element in accordance with one of claims 1 to 10 for accommodation within a magazine.

12. Use of the test element in accordance with claim 11 in a magazine, which is provided for the combined accommodation of used and unused test elements.

13. Use of a test element in accordance with claim 11 or 12 in a magazine, which is arranged within a measuring device.

14. Use of the test element in accordance with claim 13, in which the measuring device is an optical or electronic measuring device.

15. Magazine for accommodating test elements for determining an analyte in a liquid, comprising at least one test element in accordance with one of claims 1 to 10.

16. Magazine in accordance with claim 15,
**characterised in that**
it is provided for the combined accommodation of used and unused test elements.

17. Measuring device for determining an analyte in a liquid,
**characterised in that**
it contains at least one test element in accordance with one of claims 1 to 10.

18. Measuring device in accordance with claim 17
**characterised in that**
it contains at least one magazine for accommodation of one or more test elements.

19. Measuring device in accordance with claim 18,
**characterised in that**
the magazine is provided for the combined accommodation of used and unused test elements.

20. Process for determining an analyte in a liquid comprising
- Depositing of a test liquid on a test element in accordance with one of claims 1 to 10, and
- qualitative or/and quantitative determination of the analyte present in the test liquid.

## Revendications

1. Élément de test analytique pour la détermination d'un analyte dans un liquide, comprenant :
- un support inerte (1) ;
- une zone fonctionnelle pour un matériau échantillon (4) ;
- une zone de décèlement pour la détermination de l'analyte (2) ; et
- un canal ou une fente (3) pour le transport du liquide depuis la zone fonctionnelle jusqu'à la zone de décèlement ;
dans lequel l'élément de test présente, au moins dans une zone entourant la zone fonctionnelle (4), une surface à structuration hydrophobe ;
**caractérisé en ce que**
la surface à structuration hydrophobe présente un angle de contact avec des systèmes aqueux ≥ 120°.

2. Élément de test selon la revendication 1,
**caractérisé en ce que**
le canal ou la fente (3) présente une ouverture dans la zone fonctionnelle (4) et la surface de l'élément de test présente, au moins autour de l'ouverture, la surface à structuration hydrophobe.

3. Élément de test selon la revendication 1 ou 2,
**caractérisé en ce que**
le canal ou la fente (3) représente un canal capillaire ou une fente capillaire.

4. Élément de test selon l'une quelconque des revendications 1 à 3,
**caractérisé en ce que**
le canal ou la fente (3) présente à l'intérieur, au moins en partie, une surface hydrophile.

5. Élément de test selon l'une quelconque des revendications 1 à 4,
**caractérisé en ce que**
la distance moyenne entre des reliefs sur la surface à structuration hydrophobe se situe dans la plage de 50 nm à 200 µm et la hauteur moyenne des reliefs se situe dans la plage de 50 nm à 100 µm.

6. Élément de test selon l'une quelconque des revendications 1 à 5,
**caractérisé en ce que**
la surface à structuration hydrophobe présente une énergie superficielle ≤ 20 mN/m.

7. Élément de test selon l'une quelconque des revendications 1 à 6,
**caractérisé en ce que**
la surface à structuration hydrophobe peut être obtenue par pulvérisation d'une suspension de nanoparticules hydrophobes.

8. Élément de test selon l'une quelconque des revendications 1 à 7,
**caractérisé en ce que**
la surface à structuration hydrophobe est fixée sur l'élément de test.

9. Élément de test selon la revendication 8,
**caractérisé en ce que**
la surface à structuration hydrophobe peut être obtenue par l'application d'une substance durcissable sur les zones à enduire de l'élément de test, l'application de particules hydrophobes, rendues hydrophobes ou qui peuvent être rendues hydrophobes sur les zones à enduire, et la fixation des particules par durcissement.

10. Élément de test selon l'une quelconque des revendications 1 à 9, pour la détermination du glucose dans le sang.

11. Utilisation de l'élément de test selon l'une quelconque des revendications 1 à 10 pour son logement au sein d'un magasin.

12. Utilisation de l'élément de test selon la revendication 11 dans un magasin qui est prévu pour le logement commun d'éléments de test usagés et vierges.

13. Utilisation d'un élément de test selon la revendication 11 ou 12 dans un magasin qui est disposé au sein d'un dispositif de mesure.

14. Utilisation de l'élément de test selon la revendication 13, dans lequel le dispositif de mesure est un dispositif de mesure optique ou électronique.

15. Magasin pour le logement d'éléments de test pour la détermination d'un analyte dans un liquide, comprenant au moins un élément de test selon l'une quelconque des revendications 1 à 10.

16. Magasin selon la revendication 15,
**caractérisé**
**en ce qu'**il est prévu pour un logement commun d'éléments de test usagés et vierges.

17. Dispositif de mesure pour la détermination d'un analyte dans un liquide,
**caractérisé**
**en ce qu'**il contient au moins un élément de test selon l'une quelconque des revendications 1 à 10.

18. Dispositif de mesure selon la revendication 17,
**caractérisé**
**en ce qu'**il contient au moins un magasin pour le logement d'un ou de plusieurs des éléments de test.

19. Dispositif de mesure selon la revendication 18,
**caractérisé**
**en ce que** le magasin est prévu pour un logement commun d'éléments de test usagés et vierges.

20. Procédé pour la détermination d'un analyte dans un liquide, comprenant le fait de :
- appliquer un liquide échantillon sur un élément de test selon l'une quelconque des revendications 1 à 10 ; et
- procéder à une détermination qualitative et/ou quantitative de l'analyte présent dans le liquide échantillon.
